# EUROPEAN PATENT SPECIFICATION

(11) **EP 0 332 819 B1**
(45) Date of publication and mention of the grant of the patent: **01.12.1993**
(21) Application number: 89101218.9
(22) Date of filing: 25.01.1989
(51) Int. Cl.: G01N 33/94, G01N 33/577, C12P 21/00

(54) **Method for the immunoassay of dioxins and dibenzofurans**
Immunoassayverfahren von Dioxinen und Dibenzofuranen
Méthode d'essai immunologique de dioxines et dibenzofuranes

(30) Priority: 26.01.1988 DE 3802157; 29.08.1988 US 237192
(43) Date of publication of application: 20.09.1989
(73) Proprietor: THE REGENTS OF THE UNIVERSITY OF CALIFORNIA, Oakland, California 94612-3550 (US)
(72) Inventor: Vanderlaan, Martin, Dr., Danville, CA 94526 (US); Stanker, Larry H. Dr., Livermore, CA 94550 (US); Watkins, Bruce E. Dr., Livermore, CA 94550 (US); Petrovic, Peter, Dr., D-6203 Hochheim am Main (DE); Gorbach, Siegbert, Dr., D-6239 Eppstein/Taunus (DE)
(74) Representative: Goldin, Douglas Michael

(56) References cited:
- EP-A- 0 242 589
- EP-A- 0 251 635
- US-A- 4 238 472

## Description

The U. S. Government has rights in this invention pursuant to Contract No. W-7405-ENG-48 between the U. S. Department of Energy and the University of California, for the operation of the Lawrence Livermore National Laboratory.

### FIELD OF THE INVENTION:

This invention relates to an improved method for the detection of dioxins and dibenzofurans, using monoclonal antibodies and a method of sample preparation to optimize detection of dioxins by immunoassay in heavily contaminated samples.

### BACKGROUND OF THE INVENTION:

Polychlorinated dibenzodioxins (PCDD) and dibenzofurans (PCDF) are persistent, toxic pollutants which pose a threat to both human health as well as the biosphere generally. These compounds, which may also be referred to as polyhalogenated planar polycyclic aromatics, are contaminants of herbicides, such as Agent Orange, and are generated as by-products in a variety of industrial chemical processes, as well as in the course of combustion or incineration of other polychlorinated organics, such as plastics and polychlorinated biphenyls (PCB). In view of the extensive use or occurrence of such processes, dioxins and dibenzofurans are widespread in the environment. Now that the harmful nature of these materials has become fully recognized, it has become a matter of high priority to address the issue of detection of these compounds. An important, nontrivial first step is to identify sites of pollution, which requires a simple, economical, and rapid test for detection of the presence of these compounds in samples taken from industrial processing, soils, human or animal tissues, as well as foodstuffs.

Polychlorinated dibenzodioxins and dibenzofurans, and other chemicals formed during combustion and technical processes are extremely toxic and additionally environmentally stable. Efforts to monitor their formation and distribution have primarily been carried out by means of gas chromatography with mass spectroscopy, a relatively expensive procedure which involves elaborate sample preparation and the use of costly, complicated technical equipment. There is a need for a simpler and more economical analytical procedure for the substances mentioned, particularly those found in industrial samples which contain other substances which may interfere with the assay.

An important aspect of a desirable test procedure relates to specificity. There are a large number of isomers both PCDDs and PCDFs, as well as PCBs, which vary in toxicity from very highly toxic to lesser toxicities, but which are also chemically similar to other, relatively harmless compounds. A second aspect of the problem is that the most toxic isomer of these compounds in the environment, i.e., 2,3,7,8-tetrachlorodibenzo-p-dioxin, is harmful in very small amounts, and is capable of being concentrated as it moves through food chains. Hence, a desirable test must be capable of detecting the presence of these harmful substances in very low concentrations in industrial process samples, environmental samples, human or animal tissues and foodstuffs. Chemical analysis of soil samples for contamination is also hampered by several other factors: (1) dioxins bind tightly to soil and show negligible solubility in water, and (2) the multiplicity of isomers and related chemical contaminants make quantitative assay by conventional gas chromatographic and mass spectrometric methods costly and time consuming. The costs and turn-around time of assays preclude detailed sampling of an area to determine the extent of contamination, and require the use of sophisticated centralized laboratories, unsuited for field monitoring of hazardous polychlorinated dibenzodioxins and dibenzofurans.

The present invention relates to a method of preparation of samples containing dioxins and related compounds such that they are suitable for assay by immunodiagnostic methods and a test kit which is useful for this procedure. Important members of the class of polyhalogenated polycyclic aromatics with a planar structure cited are dibenzodioxins, dibenzofurans and several polyhalogenated biphenyls. For purposes of simplification, the class of substances cited shall be referred to as dioxins or dioxin, when used as an adjective, as in dioxin fraction, in the following description.

### DESCRIPTION OF THE RELATED ART:

The potential advantages of immunoassay procedures over conventional analytic methods have been recognized previously, in particular, that an immunoassay procedure offers a method which is as sensitive as gas chromatography and/or mass spectrometry, while being more rapid and less expensive.

A previous approach to detection of dioxins in the environment has been the use of a radioimmunoassay, based on polyclonal antisera produced by the immunization of rabbits with 1-amino 3,7,8,-trichlorodibenzo-p-dioxin. That method was discussed in a paper by P. W. Albro et al in Methods in Enzymology 84: 619--639 (1982) and U. S. Patent No. 4,238,472. That assay has not been widely applied because it requires the frequent synthesis of ¹²⁵I-TCDD substrate, it requires three days to complete, and because the assay uses non-specific rabbit antisera which is not sufficiently specific for the most toxic dioxin congener, 2,3,7,8-TCDD, hereinafter referred to as TCDD.

Another test for dioxins, reported by Stephen J. Kennel in a paper in Toxicology and Applied Pharmacology, 82: 256-263 (1986), is based on monoclonal antibodies produced by immunizing mice with thyroglobulin-2-adipamide, 3,7,8-trichlorodibenzo-p-dioxin has not proven specific for toxic dioxin compounds. Hybridomas produced by spleen cells of animals immunized to this compound and fused to myeloma cells will secrete antibodies which have inadequate selectivity for nonprotein conjugated 2,3,7,8,tetrachlorodibenzo-p-dioxin in solution, the most toxic of the dioxin isomers, and other dioxin isomers, while they do react with compounds which are not toxic dioxins. The test also uses a radioimmunoassay procedure with its attendant disadvantages.

Detection of dioxins discussed in recent publications describe immunoassays which employ monoclonal antibodies which bind specifically to toxic dioxins. The publications are: Stanker, L., Watkins, B., Rogers, N. and Vanderlaan, M., "Monoclonal Antibodies to Dioxin, Antibody Characterization and Assay Development", J. Toxicol., 45: 229-243 (1987); and Stanker, L., Watkins, B., Vanderlaan, M., and Budde, W., "Development of an Immunoassay for Chlorinated Dioxins Based on a Monoclonal Antibody and an Enzyme Linked Immunosorbency Assay (ELISA)", Chemosphere 16: 1635-1639 (1987)). Chemically pure dioxins, or samples from which the dioxin may be readily isolated, can be readily analyzed by those procedures, however, samples from industrial processes, which contain other substances which may be interactive with the assay, were not tested. Until this invention, a procedure for analysis of industrial and environmental samples has not been available because of difficulties which are encountered during sample preparation which require partial purification of the dioxin fraction and then solubilization of the dioxin fraction into an aqueous solution for specific antibody detection. The procedure herein described enables the use of immunodiagnostic assay of dioxins in samples which may contain interfering contaminants of chemical processing or environmental samples.

Previous investigators have used various preparative extraction and chromatographic techniques for samples to be evaluated for dioxin contamination. These techniques are suitable for preparation of samples to be analyzed by gas chromatography/mass spectroscopy. Such prepared samples may still have components which interfere with immunoassays. Preparative methods include multiple chromatography treatment of samples for GC/LRMS analysis, alumina columns to partition dioxins from materials found in a hazardous waste storage site and Soxhlet extraction with phenol or toluene for dioxins, although these gave a wide variation in results, and the procedure was unsuitable for fly ash. Multiple extraction methods have been used to identify very minute dioxin contamination by GC/MS analysis and sequential solvent extraction for HRGC/MS analyses. All of these described procedures were used to prepare samples for GC/MS analysis. It was not determined whether those samples were free of substances which may interfere with antibody reactions which take place in an aqueous environment. The procedures previously described using non-ionic detergents indicated that only Triton® X-305 and Cutscum™ were suitable in radioimmunoassays, because other detergents did not solublize the TCDD, they "shielded" it from the antibody or they interacted with antisera proteins. It was not distinguished whether these detergents would interfere in reactions using dioxin-specific monoclonal antibodies.

In a comparison of interlaboratory test reliability and sensitivity, it was determined that the practical limits of sample detection by GC/MS on samples prepared from technical grade products was about 10ppb, with a reproducibility of +/- 100%. Application of the herein described method of sample preparation and analysis by means of monoclonal antibodies yield a similar or better degree of detection and reproducibility.

### OBJECTS OF THE INVENTION:

The foregoing clearly indicates that there exists a continuing need for an effective, practical test for detection of toxic dioxins.

Accordingly, a major object of this invention is to prepare samples from industrial or chemical processes for immunoassay of dioxins, such that the assay will have the necessary selectivity and sensitivity to conclusively indicate the presence of dioxins in concentrations of a few parts per billion (nanograms per sample).

Another objective is to provide a simplified extraction and detection test for dioxins which can be carried out rapidly in the field environment.

Yet another object is to provide an immunoassay capable of conclusively distinguishing the most toxic of the dioxins.

Another object is to define a procedure whereby sensitivity of dioxin detection can be raised to achieve detection at a few parts per trillion level.

Another object is to provide qualitative and quantitative determination of polyhalogenated polycyclic aromatics with planar structure, which are referred to as dioxins, in crude industrial and environmental samples, including soils.

### SUMMARY OF THE INVENTION:

The present invention is a method for preparation of samples for determination of the presence of polychlorinated dioxins and particularly 2,3,7,8-tetrachlorodibenzo-p-dioxin (TCDD), using monoclonal antibodies which are obtained from hybridoma cell lines. The production of the cell lines and characterization of the antibodies derived therefrom has been described in greater detail in EPO Application Publication No. 251635 by Vanderlaan et al. These antibodies are characterized by high affinity and selectivity toward TCDD. Their use with an improved ELISA test protocol (Enzyme-linked-Immunosorbent-Assay) permits the determination of the presence of dioxins in test samples at levels of a few parts per billion and less. Tests can be completed in hours rather than days. The use of radio-labeled compounds is obviated. Antibody tests for TCDD can be conducted in aqueous media with solubilization of the organic test compounds increased by detergents and/or sonication. In a preferred mode, the sensitivity of the immunoassay procedure can be raised through solubilization of the sample material by addition of detergents at a concentration on the order of about 0.01-2.0% by weight. Use of limited concentration of coating antigen and amplification of the peroxidase enzyme endpoint with a avidin/biotin amplification system makes it possible to detect TCDD concentrations in the range of a few parts per trillion. Other antibody preparations obtained using desired dioxins as immunogens can also be used.

The sample preparation procedures described herein enable analysis of industrial samples, which may include reaction mixtures, distillation residues, chemicals, fly ash, filter dust, transformer fluid (PCB oil) or motor oil, by detection methods with greater sensitivity. Additionally, the procedure may be employed for examination of samples of other origins, including environmental air, water and soil samples, human and animal tissues, and foodstuffs. The sample preparation procedure includes preliminary extraction and separation of the fraction containing the dioxin compounds of interest and subsequent solubilization of that fraction with detergents to expose the dioxin compounds to the aqueous immunoassay reactants. Selection of an appropriate antigen coating concentration of the reaction vessel and selection of an enzymatic amplification system for the ELISA will further increase the sensitivity of the assay. The method is particularly suited for analytical determination of polychlorinated dibenzodioxins and dibenzofurans, and specific detection is effected into the range of a few parts per trillion.

### BRIEF DESCRIPTION OF THE DRAWINGS

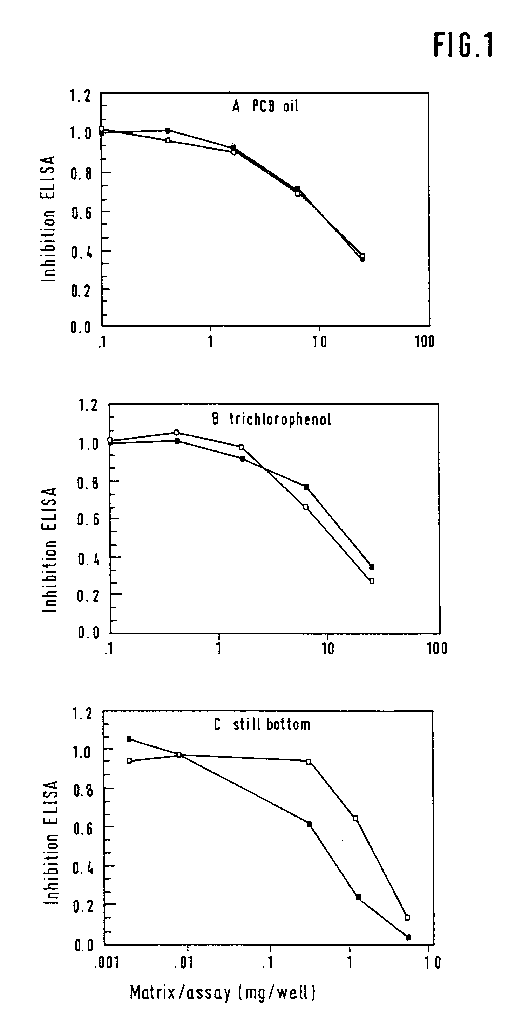

Figure 1 - Inhibition ELISA data is shown for sample extracts prepared by clean-up of carbon column alone (open square) or carbon column followed by oleum-silica column (closed square). Additional sample extraction with oleum-silica column did not alter I₅₀ on samples of PCB oil (panel A) or trichlorophenol (panel B), but the I₅₀ was lower for still bottom sample # 1 (panel C), a distillation residue from a chloronitrobenzene still, indicating that the second extraction did remove non-dioxin, immunologically cross-reactive material.

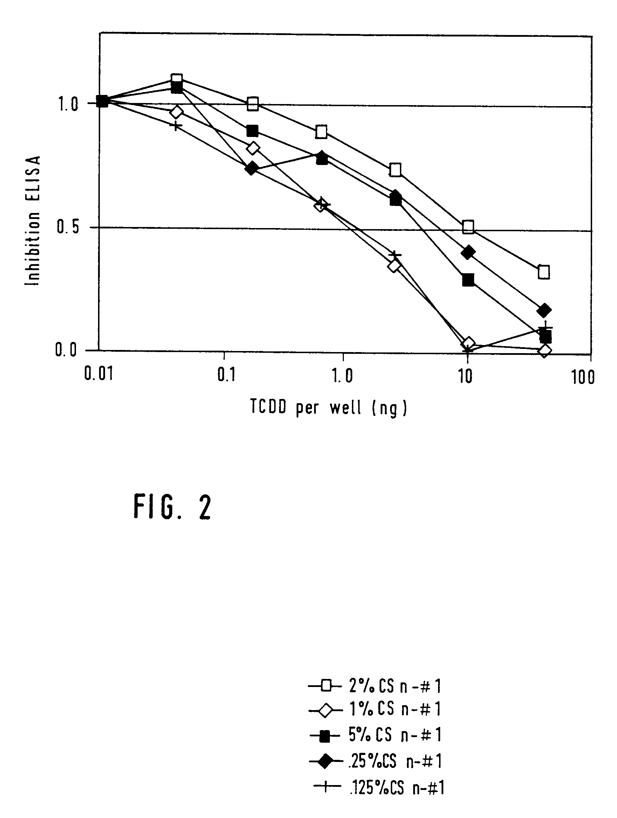

Figure 2 - Sensitivity of dioxin immunoassay is improved with detergent, Cutscum™, solubilization of TCDD standard. Optimal assay sensitivity (I₅₀) is achieved with 0.25% (open diamond) and 0.125% (cross) Cutscum™, whereas higher detergent concentrations, 0.5% (closed square); 1.0% (closed diamond) and 2.0% (open square) progressively inhibited competition. Detergent concentrations less than 0.1% failed to solubilize dioxin.

### DESCRIPTION OF THE INVENTION:

### Source of Antibodies

The present method of sample preparation will produce samples which can be effectively analyzed for dioxins with monoclonal antibodies which are specific for polychlorinated dibenzodioxins (PCDD) and dibenzofurans (PCDF). Antibodies which were raised to discriminate the functional groups of dioxin molecules will have adequate specificity in the assay described herein.

Hybridomas which produce polyhalogenated planar polycyclic aromatic-specific antibodies, referred to as dioxin specific antibodies, are used. Illustrative are the named hybridomas, DD-1, DD-3, DD-4, DD-5, and DD-6, described by Vanderlaan, et al in USSN 877,909 published as US-A-4 798 807 and equivalent to EP-A-0 251 635. These hybridomas are publicly available in the depository of the American Type Culture Collection (ATCC), Rockville, Maryland, and are identified with the following accession numbers: DD-1, (HB 9741); DD-3, (HB 9742); DD-4, (HB 9743); DD-5, (HB 9744); and DD-6, (HB 9745). These cells were produced by fusion of myeloma cells with splenocytes obtained from a mouse immunized with the TCDD analog, 1-amino-3,7,8,-trichlorodibenzo-p-dioxin (A-triCDD). This compound was synthesized and then conjugated to thyroglobulin carrier protein. After immunization with the hapten-protein complex, the immunoreactive spleen cells were collected and fused with myeloma cells, and the resulting hybridomas were screened for those with the ability to recognize TCDD, which was bound to protein and to free suspended TCDD. From these screenings, five hybridomas (named DD-1, DD-3, DD-4, DD-5 and DD-6) were selected for further study of their specificity. The specificity of these antibodies for a variety of dioxins, dibenzofurans, PCBs and other chlorinated hydrocarbons is reported by Vanderlaan, et al. in EPO Application Publication No. 251635. The binding specificities of these antibodies are highly desirable, since they bind selectively to the most toxic of the dioxin and dibenzofuran isomers.

### Sample Preparation

The analytical procedure suitable for industrial and environmental samples was developed to overcome problems of real world sample analysis by initiation of a partial purification and extraction of the toxic dioxins prior to their solubilization into aqueous solutions for immunoassay. The sample preparation procedure enables qualitative and quantitative determination of dioxin compounds in industrial samples of reaction mixtures, distillation residues and other heavily contaminated samples. Monoclonal antibodies specifically reactive with dioxins were described above (Stanker, et al (1987) J. Toxicol., 45: 229-243). As individual antibodies may react with certain compounds or groups of compounds, the results of several preparative schemes are best compared by relating to a standard compound of known concentration in a standard sample.

It is expedient to extract and/or prepare industrial samples before conducting the immunoassay reaction for determination of dioxins. This is accomplished, according to the method of the invention, by the following consecutive steps:
a. Preparation of a raw extract by decomposition of the sample via acid treatment,
b. Separation of the fraction of polyhalogenated polycyclic aromatics with a planar structure, referred to as the dioxin fraction,
c. Solubilisation of the dioxin fraction into an aqueous buffer solution. These steps are then followed by assay of the digoxin fraction thus obtained by means of reaction with specific antibodies.

It is best to perform acid extraction prior to analysis of primarily inorganic samples such as fly ash, filter dust and inorganic chemicals. Various acids are suited to this end, in particular concentrated HCl and H₂SO₄ being preferred. Following acid treatment, the mixture is then extracted with an organic solvent or a mixture of solvents. Various organic solvents are suited to this end. The preferred solvents Include CCl₄, CHCl₃, CH₂Cl₂, benzene, toluene, chlorobenzene, chlorotoluene, hexane, cyclohexane, chlorocyclohexanes, methanol and ethanol. Especially preferred are CH₂Cl₂ and cyclohexane, in particular, mixtures of these two solvents.

Slightly soluble samples which are primarily organic in nature, such as reaction and distillation residues, are preferably prepared by suspending them in an acid, preferably concentrated H₂SO₄ saturated with SO₃, and then mixing them with a solid (e.g. silica gel), preferably until the mixture becomes flowable. This mixture may subsequently be extracted at boiling heat in a suitable apparatus, preferably with one of the solvents cited above or with a corresponding mixture.

Readily soluble samples, which are primarily organic in nature, such as motor oil, PCB oil and organic chemicals, are advisedly extracted, without previous acid treatment, with one or more of the solvents mentioned above. Preferred here is extraction in the boiling heat of the solvent in question (e.g., using a Soxhlet extractor).

The dioxin fraction may be separated in various ways from the extract obtained. To be preferred here is separation by means of a chromatographic procedure similar to that described by the Environmental Protection Agency in "Polyhalogenated Dibenzo-p-Dioxins/Dibenzofurans; Testing and Reporting Requirements. Federal Register, 52: 108, June 5, 1987, pp. 21412-21452, 40CFR parts 707 and 766. Especially preferred is separation by means of solid-liquid chromatography, for example with a solid phase consisting of glass fiber homogeneously mixed with active carbon. The glass fibers and the active carbon are advisedly blended (e.g., with a device having counter-rotating blades or with a mill) if necessary following the addition of a solvent. The content of active carbon measures about 5 to 15% by weight, with a preferred content of about 8-12%, especially about 10%, being preferred in particular.

The dioxin fraction is best separated with the latter method in a column procedure. To this end, the dioxin extract is applied to the column provided with a solid phase of glass fibers coated with active carbon. The column is washed following the application of the dioxin extract, if necessary, preferably with a mixture consisting of CH₂Cl₂ and hexane, with a mixture ratio of about 0.8 to 1.2 : 0.8 to 1.2 being especially preferred. Another washing procedure with other solvents may follow, with a mixture consisting of dichloromethane, methanol and toluene being preferred, especially in a mixture ratio of about 65 to 85 : 15 to 25 : 0 to 10. Many other solvents and mixtures thereof are also suited for the washing procedures described. Preferred solvents are those which were already cited in connection with manufacture of the extracts. The optimum composition of the washing liquids may have to be determined empirically for special extract compositions.

The dioxin fraction is preferably eluted after inverting the column 180 degrees and various solvents or mixtures thereof may be employed for this process. Preferred here is the application of toluene, benzene, xylenes and their chlorinated derivatives. Toluene is particularly preferred. The determination of suitable compositions for the washing and elution liquid may be simplified via experiments in which a marker dye is added to the dioxin extract. The marker dye (e.g., fat blue B (p-anthraquinone derivative manufactured by Hoechst AG, Frankfurt/Main)) is adsorbed and eluted in a similar manner to the dioxin fraction in question. Marker dyes such as these are also suitable for monitoring the efficacy of individual washing steps of the elution process.

An additional cleansing operation may be used in exceptional cases where the dioxin fraction still contains interfering components after a single chromatographic wash. This may be another solid-liquid chromatographic extraction, for example. Chromatography using a column with acid-impregnated silica gel as the solid phase has been shown to be especially well suited. This column may be made by addition of about one part of SO₃-saturated H₂SO₄ to approximately 8 to 12 parts of oleum-saturated silica gel and heating this mixture for about 10 to 20 minutes to about 70-90 °C. The still impure dioxin fraction is added to one part of the activated silica gel, and this mixture is poured into a column as the uppermost layer. Additional layers consisting of about 5 to 15% AgNO₃ silica gel (obtained by mixing an aqueous AgNO₃ solution with silica gel and subsequently evaporating the water) and of oleum silica gel may be poured in. The layers should be separated from one another by silica gel or activated silica gel. The dioxin may be obtained from a column manufactured in the manner described via elution with a non-polar organic solvent such as hexane, cyclohexane, benzene and toluene, preferably hexane. The ELISA assay data of Figure 1 illustrate that a second extraction with the oleum-silica column did not alter the immunoassay analysis results following a single carbon column extraction for dioxin congeners in PCB transformer oil (panel A) or in trichlorophenol (panel B), but did remove non-dioxin, immunochemically cross-reactive material from the distillation residue of still bottom sample # 1 (panel C).

The dioxin fraction obtained as per the method described is then assayed for the detection of dioxins by reaction with monoclonal or polyclonal antibodies. The immunoassay may be applied in various ways, for example as described in P. Tijssen, " Practice and Theory of Enzyme Immunoassays" (R.H. Burdon and P.H. Van Knippenberg, eds.) Elsevier, Amsterdam (1985). The immunoassay is preferably applied as a competitive assay. To this end, the concentrated dioxin fraction is best dissolved in an organic solvent or mixture of solvents, with non-polar organic solvents such as hexane and cyclohexane being particularly suited for these purposes. Advisedly added to this solution is an alcohol solution of detergents, preferably one or more nonionic surfactants such as "Cutscum"™ (isooctylphenoxy-polyethoxyethanol, Fischer Scientific Company, Raleigh, NC, USA), polyoxyethylene ethers such as polyoxyethylene stearyl ether or polyoxyethylene lauryl ether, N-octanoyl-glucopyranosides, N-heptylglucopyranosides, nonanoyl-N-methylglucamide, heptanoyl-N-methyl glucamide, polyoxyethylene sorbitan monolaurate, octylphenol ethoxylate, 3-(3-cholamidopropyl)-dimethylammonio-2-hydroxy-1-propane sulfonate, 3-(3-cholamidopropyl)-dimethylammonio-1-propane sulfonate or octanoyl-N-methyl glucamide, with polyoxyethylene ethers such as polyoxyethylene stearyl ether and polyoxyethylene lauryl ether, polyoxyethylene sorbitan monolaurate, which is known as Tween™, octylphenol ethoxylate, and "Cutscum"™ being preferred, especially "Cutscum"™.

The detergents mentioned are preferably employed in a concentration of about 0.01 to about 2 percent by weight, with a preferred concentration of less than 1 percent by weight, especially of about 0.1 to 0.4 percent by weight , being preferred in particular. The effect of detergent concentration on immunoassay sensitivity is demonstrated in Figure 2. The maximum effectiveness occured when the detergent concentration is at about 0.1 to 0.4 percent by weight. When the concentration of detergents is too high, the dioxin dissolves well but is no longer "recognized" by the antibody, which results in reduced sensitivity of the immunoassay. This aqueous solution of detergents may also contain a portion of organic solvent such as acetone or methanol to ease pipetting.

The combined solutions are advisedly dried, for example, via a stream of nitrogen or warm air. The monoclonal antibody, which may be dissolved in an aqueous buffer solution, is subsequently added.

The aqueous buffer solution for suspension of the dioxin fraction is comprised of various substances. It preferably comprises the following: about 0.05 to 0.15 moles per liter of a compound or mixture of compounds buffering in a pH range of 7 to 8, with buffers based on tris(hydroxymethyl)-aminomethane or phosphate buffers being preferred in particular; about 0.1 to 5% by weight of a protein, especially about 0.2 to 1%, of a protein, preferably egg albumin, rabbit serum albumin or bovine serum albumin, with bovine serum albumin, especially preferred; about 0.1 to 0.3 moles per liter of an alkali halide, preferably NaCl; about 0.005 to 0.5 % by volume of detergents, especially preferred 0.008 to 0.012 percent by volume of detergents, preferably one of the detergents mentioned above, in particular polyoxyethylene sorbitan monolaurate.

### Immunoassay

The preferred vessel for execution of the competitive immunoassay is an antigen-coated test tube, preferably consisting of polystyrene, preferably in the form of a microtiter plate. The coating antigen may be applied in various ways, for example as described in the aforementioned monograph of R. Tijssen. Preferably employed is a solution of dioxin-protein conjugate consisting of polyhalogenated polycyclic aromatics having a planar structure, preferably dibenzodioxin or dibenzofuran, reacted with mammalian serum albumin, preferably rabbit or bovine serum albumin, with which the reaction vessel is treated.

After the coated vessel has been incubated with an antibody-dioxin mixture, the unbound material may be rinsed out and the bound antibody material detected in a known manner, for example by measurement of the labeling of the antibody. A radioactive isotope or compounds which may participate in a chemiluminescence or fluorescence emission in an enzyme-catalyzed reaction may be used for labeling. The larger the detected signal during the competitive assay, the lower the dioxin concentration in the tested sample. Previous operation of the assay demonstrated a practical limit of hapten detection when the amount of protein-hapten conjugate used was limited to 50 ng/well.

However, a preferred method for execution of the competitive immunoassay includes coating the immunoreactive wells with a limited amount of antigen, as less analyte is required to draw the antibody from the bound hapten. Optimal sensitivity of the detection system is obtained when the preferred concentration of the plating antigen is reduced from 100 ng to about 0.5 ng of coating antigen per well, a concentration of between about 0.2 ng to 1.0 ng being especially preferred. To detect such a limited quantity of coating antigen, however, the signal must be additionally amplified. A preferred method of amplification of the enzyme-catalyzed optically-detected signal is with an avidin-peroxidase/biotin-anti-mouse immunoglobulin system which improves the sensitivity of the immunoassay when run with low background levels of plated antigen. The avidin-peroxidase/biotin-anti-mouse immunoglobulin system may be used to improve the efficacy of enzyme-linked immunoassay as the biotin can be easily coupled to antibodies or enzymes without loss of activity. The exceptionally high binding affinity of avidin ( 10¹⁵ /M) for biotin results in the formation of bridging complexes between biotinylated molecules. Both the specific-binding monoclonal antibody and the peroxidase-enzyme indicator system are bound to biotin. Linkage of the biotin molecules with avidin results in complexing of the indicator molecules and increases the sensitivity of the spectrally detectable signal.

Dioxin specific antibodies may be conjugated to biotin by the method described in Chapter 3 of the aforementioned P. Tijssen monograph. Biotinyl-N-hydroxysuccinimide (BNHS) ester can be reacted with the dioxin specific antibodies to make the biotinylated immunoreactants. In a preferred method, the dioxin-specific antibody can be identified by the binding of a biotinylated anti-mouse IgG immunoglobulin to the antibody. Biotinylated peroxidase, Vectastain (Vector Laboratories, Burlingame, CA) can be used as the detection signal after bridging to the avidin-biotin amplification complex.

A statement of the dioxin contamination of a sample may be made with a single analysis using the procedure described in this invention. In order to ensure a higher level of certainty of quantitative statements, however, it is best to measure a dilution series of the sample under examination, preferably with contemporaneous standard samples of known dioxin content. Radiolabeled dioxin was used to determine the efficacy of the various methods used to suspend dioxin in the aqueous assay solutions. Sample recovery efficiencies were typically about 90%, with not less than 70% recovery.

A test kit can be assembled for execution of the reaction as per the invention which contains all necessary chemicals, such as chromatography material solvents and eluents, test tubes, detergents, antibodies and chemicals for the detection reaction. Such a test kit should preferably be based on endpoint determination by fluorescence reaction or an enzyme-catalyzed reaction, because the outlay of equipment for the test reaction kit would then be economical and conveniently available for use in almost any laboratory.

### EXAMPLE 1 - Extraction Procedure

Dioxin content of a distillation residue of chlorinated aromatics may be examined with specific monoclonal antibodies following extraction and fractionation. A 5 g sample of distillation residue is suspended in 20 ml of H₂SO₄ saturated with SO₃ and mixed with silica gel until a flowable mixture is obtained. The mixture is extracted in a Soxhlet extractor with approximately 200 ml of boiling toluene. The rotary evaporator extract and 1 mg of fat blue B are dissolved in 50 ml of dichloromethane/cyclohexane (50:50) and loaded into a column filled with 1 g of active carbon (10%) on glass fibers (63-200 µm size), which have been activated by drying at 130°C for several hours. The column is washed with 50 ml of dichloromethane/cyclohexane (50:50) and 75 ml of dichloromethane/methanol/toluene (75:20:5). The column is subsequently inverted and eluted in the opposite direction with 50 ml of toluene. The toluene eluate contains the "dioxin fraction" and is dyed blue. The toluene is removed by rotary evaporation and the residue is dissolved in 1 ml of n-hexane. The hexane solution is loaded on 2 g of oleum/silica gel (1:10), which has also been activated by previous heating to 130 °C, and mixed in the rotary evaporator for 15 minutes at 80°C. The sample which has adsorbed on the silica gel is loaded into a column already filled with 1 g of oleum/silica gel (1:10), 1 g of silver nitrate/silica gel (1:10) and 0.5 g of silica gel between each layer. The column is eluted with 50 ml of n-hexane. The eluate containing the "dioxin fraction" is evaporated in the rotary evaporator, the residue dissolved in 500 µl of n-hexane and examined for dioxin content in the competitive immunoassay. By competitive immunoassay, the sample evaluated had a content of 2,3,7,8-TCDD equivalents measuring 25 ppb.

### EXAMPLE 2 - Immunoassay Procedure

The competition test procedure for dioxins has been discussed in detail by Stanker, el al. (1987) (Chemosphere 16: 1635-1639). A large part of the success of the instant method, which uses monoclonal antibodies with characterized binding specificity, is attributed to the development of a reliable competition ELISA protocol. Microtiter plates are coated with tri-CDD-albumin (antigen) solution, exposed to a blocking solution of albumin and then subjected to a dilution series of solubilzed sample or dioxin standard. When the antibody solution is added, the antibody partitions between the tri-CDD-albumin (antigen) bound to the plate and to the dioxin sample in solution. After reaction, the sample solution is removed, and the plate is rewashed and incubated with an enzyme-tagged identification molecule. Development of the enzyme-antibody conjugate with appropriate substrate permits visualization of the monoclonal antibody which has bound to the tri-CDD-albumin (antigen) that is fixed to the plate. The results are then expressed as a fraction of the response in wells with no competitor. From such data, one can determine the concentration required to inhibit antibody binding by 50% (I₅₀).

For the immunoassay, which has been described by Stanker, et al (1987), the "dioxin fraction" is divided into five equal parts of 100 ul. Two parts remain unchanged, while one part is diluted six times at a ratio of 1:2, and two parts are spiked with 1 or 10 ng of 2,3,7,8-TCDD standard. Detergent solution, 40 µl of 0.5% Cutscum™ in acetone, is added to each sample, and the solvents are removed under a stream of nitrogen. The residues are resuspended in the tris-hydroxymethyl (aminomethane) buffer with 100 µl of the dioxin specific antibodies (100 ng/ml) and applied to a polystyrene microliter plate coated with trichlorodibenzodioxin(tri-CDD)-rabbit serum albumin conjugate. The retained antibodies are quantitatively determined with sheep anti-mouse IgG peroxidase and o-phenylene diamine(OPD) substrate in a dye reaction, with light absorption measured at 492 nm.

A preferred method for the immunoassay includes amplification of the peroxidase enzyme endpoint by coupling to a avidin-peroxidase/biotin-anti-mouse immunoglobulin complex. Optimal enzyme sensitivity occurs when a minimal amount of coating antigen (tri-CDD-BSA) is used. The total protein binding capacity of the plate is large so the extra unconjugated carrier protein does not degrade binding. In the preferred method, microtiter plates are coated with a tri-CDD-BSDA solution by addition of about 0.2 - 1.0 ng of tri-CDD-BSA/well, more preferably, 0.5 ng/well. The coated plates may be dried and stored until needed.

Sample extractants are transferred from vials with hexane to antigen-coated microtiter wells, where they are solublized with 40 µl of 0.5% Cutscum™ detergent/acetone. Samples are dried overnight at room temperature or with a stream of blown hot air at 45° C. The immunoassay is run by incubation of samples with dioxin-specific antibody at a concentration of 100 ng/ml in assay buffer for 1 hr at 37 °C. The microtiter plates are washed (2X) with a 0.5% detergent (polyoxyethylene sorbitan monolaurate) wash solution.

The antibody which has partitioned between the bound dioxin antigen at the plate wall and the unbound dioxin of the extracted sample is visualized when bound to the plate-wall antigen by binding with biotinylated anti-mouse IgG immunoglobulin (approximately 1.5 µg/well After a 45 minute incubation, avidin-peroxidase complex and biotinylated anti-mouse immunoglobulin are added and reacted for another 30 minutes. Following a 5X wash with 0.5% detergent (polyoxyethylene sorbitan monolaurate) solution, peroxide and ortho-phenlyenediamine (OPD) substrate are added and an initial Fast ELISA slope reading made at 450 nm and final Fast ELISA readings made at 492 nm, following stop with concentrated H₂SO₄.

Sample values are compared to a standard curve run on each microtiter plate. The standard curve shows a 50% inhibition value between 0.1 and 0.3 ng of TCDD. Normalized data is reported as a function of gram-equivalents of starting material per well.

A serial 1:2 dilution of a 2,3,7,8-TCDD standard, in the 0.1 to 500 ppb range, is carried out at the same time on each microliter plate.

### EXAMPLE 3 - Evaluation

A sample concentration may be reported in TCDD equivalents based on the standard curve made by serial dilution of the 2,3,7,8-TCDD standard. The doubled value of the undiluted sample should correspond to the values in the series subjected to dilution. An interference caused by matrix components which may not have been removed is indicated by discrepancies in the spiked samples. A sample measurement is invalidated if the concentration ascertained in the spiked samples does not correspond to the sample plus the added 1 or 10 ppb of the spiked sample.

### Example 4 - Industrial Process Samples

A variety of industrial chemical samples derived from laboratory and technical processes were evaluated for the contamination of dioxins and dibenzofurans present by the above-described method (Table I at end of specification, page 37). The demonstration assays include five samples of various distillation residues (still bottom), 2,4,6-trichlorophenol (TCP), nitrodiphenylether, a fly ash sample, and a sample of polychlorinated biphenyl (PCB) oil from a transformer which had been involved in a fire. These samples varied considerably in kind and content of contaminants present. The contamination level of dioxins determined in samples prepared for immunoassay were compared with dioxin contamination of parallel samples assayed by gas chromatography/mass spectroscopy provided by Dr. B. Bogdoll (Anders, H., A. Buhlmann and B. Bogdoll - unpublished results). The GC/MS data was reported as the total content of all tetraand penta-chloro congeners, and as the total contamination of the four most toxic congeners, (2,3,7,8-tetraCDD and 2,3,7,8-tetraCDF, and 1,2,3,7,8,-pentaCDD and 2,3,4,7,8-pentaCDF). Most samples contained a mixture of PCDFs and PCDDs while some contained only one of these groups of compounds. Samples were also included which had no history to suggest PCDD and PCDF contamination. These included technical grade 3,4-dichloronitrobenzene (dCNBz) which was thought to possibly cross-react with the antibodies; paraffin, which is not easily evaluated by GC/MS analysis; and HD-30 machine oil, which is a common matrix that is analyzed for PCDD.

ELISA analyses of chemical process samples required different amounts of assay material. The amount of starting material needed for determination of the 50% inhibition point of the ELISA was 1 mg for still bottom sample #1, 10 mg each for samples of PCB and TCP, and 0.4g for the fly ash sample. These samples demonstrated a contamination level of 5,000, 500, 500, and 25 ppb levels, respectively, when expressed as equivalents of TCCD. Identical estimates of TCCD contamination were made when these samples were reanalyzed by immunoassay on a subsequent day.

Four samples which did not contain agents which would detectably inhibit the ELISA assay, the two dCNBz samples, paraffin, and machine oil, were evaluated to determine if these matrices interfered with the dioxin detection assay. Samples spiked with 1, 3, or 10 ng of tetraCDD, showed similar inhibition of the ELISA as would be caused by addition of similar amounts of dioxin in hexane.

### EXAMPLE 5 - Soil Samples

Soil samples were evaluated in several extraction procedures in an effort to determine a minimum process time which would yield high recovery of dioxins. Comparisons made on procedures using both the large-hexane-volume overnight-extraction protocol or a shorter extraction protocol with two washes of hexane showed that they each yielded greater than 90% recovery of an added radiolabeled spike of TCDD.

A rapid-extraction method that used two successive 10 ml hexane washes of the soil-sodium sulfate sample, without overnight mixing was evaluated. A lower and more variable recovery of the radiolabeled tracer was observed when the extraction method used acetonitrile:methylene chloride (1:1).

In an exemplary extraction procedure for soil samples, 10 grams of wet soil was dried by shaking with 10 grams of sodium sulfate for 1 hour. The sample was extracted with 10 ml of hexane and 5 ml of ethanol centrifuged and the hexane layer removed by pipet. The mixture was re-extracted with 10 ml of hexane which was mixed and shaken for 15 minutes. Vigorous shaking, via a commercial paint shaker, prior to separation of the phases improved the efficacy of the extraction. The hexane layers were combined and reduced in volume in a rotary evaporator. The solvent was removed under a dry nitrogen gas stream and the residue was resuspended in 1 ml of 1:1 dichlorohexane:cyclohexane and spiked with fat blue B. The sample was applied to a column of activated carbon/glass fibers (1:9), the column was washed with dichloromethane/methanol/toluene (15:4:1) and the dioxins were recovered by reverse elution with toluene. The PCDD's (polychlorinated dibenzodioxins) and PCFD's (polychlorinated dibenzofurans) co-eluted with the colored fat blue B marker. The sample was then applied to a column of fuming sulfuric acid impregnated silica gel/silver nitrate impregnated silica gel, eluted with hexane, and evaporated. The sample was solubilized as described previously and analyzed by the immunoassay.

Soil samples spiked with 0.1 - 10ppb of tetrachlorodibenzodioxin (TCDD) typically showed 95% recovery of the TCDD spike in the extraction washes. Extracts of composite soils, taken from 0.1 and 1.0g of soil, did not interfere with a TCDD standard curve run in buffer. When spiked soil samples were extracted by the above method and analyzed in an assay which corresponded to 0.2 g equivalents of soil/well, the immunoassay results corresponded well with the expected values for soils contaminated with dioxins in the range of 0.1 - 10 ppb. There was a greater correlation of immunoassay results with the spiked sample amount when 1.0 g equivalent of soil extract/well was assayed. This gave linear correlation in the 0.1 to 1.0 ppb range, although assays of samples of extracts at 10 ppb were outside of the linear region of the curve.

Multiple analyses of a soil sample were consistent when analysis was repeated over a period of several months. A selected soil sample, with an organic content of less than 1% and a sand content of 80%, was extracted overnight in hexane, followed by an overnight extraction with sulfuric acid. The sulfuric acid treatment did not interfere with the immunoassay. The inhibition curve obtained with soil extract and in spiked samples of soil extract indicated that there were detectable levels of dioxins present in the soil extracts.

Residual matrix materials of various soils may interfere with the assay. Even after extraction, some aspect of some soils continued to sequester the dioxin, making it unavailable to the dioxin-specific antibodies. Some soils have large quantities of hexane soluble organic material to which dioxin binds instead of partitioning into the solvent used in the immunoassay. With some samples, preliminary alumina or silica column chromatography did not improve the sensitivity of the assay. The total organic content of the soil or the physical properties of soil were not,alone, good indicators of whether the soil could be suitably analyzed for dioxin by immunochemical means. For some soils of low organic content, simple extraction in hexane, with sulfuric acid treatment will effectively remove interfering matter, such that immunoassay can detect dioxins contamination in the range of 1 ppb. The above-described preparative conditions are expected to be equally suitable for tissue or food samples.

**TABLE I**

| Chemical Samples Analyzed for Dioxins | | | | | |
|---|---|---|---|---|---|
| Sample | | | Contamination Level (ppb) | | |
| No. | Type | Size^{a} (g/assay) | GC/MS^{b} | | Immunoassay |
| | | | All tetra and penta chloro congeners | Most toxic congeners | TetraCDD equivalents |
| 1. | Still Bottom | 0.005 | 18.000 | 1725 | 5.000 |
| 2. | Still Bottom | 0.100 | 550 | 260 | 125 |
| 3. | Still Bottom | 5.000 | 1.1 | 0 | 0.6 |
| 4. | Still Bottom | 0.100 | 50^{e} | 0.3 | 250 |
| 5. | TCP | 0.025 | 2.600 | 125 | 500 |
| 6. | nitrodiphenylether | 0.100 | 21^{f} | 0 | 500 |
| 7. | PCB oil^{c} | 0.025 | 1.700^{g} | 290 | 500 |
| 8. | Fly ash | 2.000 | 272 | 14 | 25 |
| 9. | 3.4-dCNBz | 0.050 | <1 | <1 | <20 |
| 10. | 3.4-dCNB | 0.050 | <1 | <1 | <20 |
| 11. | Paraffin | 0.050 | <1 | <1 | <20 |
| 12. | Machine oil^{d} | 0.050 | <1 | <1 | <20 |
| 13. | Still Bottom | 1.000 | 19 | 7 | 25 |

| | | | | | |
|---|---|---|---|---|---|
| ^{a}Largest amount of sample extract tested per well in the ELISA. Expressed in terms of mass-equivalents of starting material. | | | | | |
| ^{b}GC/MS date provided by Dr. Bogdoll [12]. The "Most Toxic Congeners" numbers are the sum of 2,3,7,8-TetraCDD, 1,2,3,7,8-pentaCDD, e,3,7,8-TetraCDF, and 2,3,7,8-pentaCDF. | | | | | |
| ^{c}From a transformer that had burned. | | | | | |
| ^{d}HD-30. | | | | | |
| ^{e}Sample contained dioxins only. | | | | | |
| ^{f}Sample also contained 18,000 ppb of TriCDD. | | | | | |
| ^{g}Sample contained furans only. | | | | | |

## Claims

1. Method for qualitative and/or quantitative determination of polyhalogenated planar polycyclic aromatics, referred to as dioxins and dibenzofurans in a sample comprising:
a) preparation of a raw extract by decomposition of the sample via acid treatment;
b) separation of the fraction of polyhalogenated polycyclic aromatics with a planar structure known as the dioxin fraction;
c) solubilization of said dioxin fraction into an aqueous solution; and
d) assay of the dioxin fraction thus obtained by means of reaction with specific antibodies.

2. Method as claimed in claim 1, wherein the dioxins and dibenzofurans are determined in industrial samples.

3. Method as claimed in claim 1, wherein the dioxins and dibenzofurans are determined in environmental samples.

4. Method as claimed in one or more of the preceding claims wherein the specific antibodies are monoclonal antibodies.

5. Method as claimed in claim 4, wherein the specific antibodies are selected from hybridomas of the group comprising DD-1, DD-3, DD-4, DD-5 or DD-6, with ATCC Accession Nos. (HB 9741), (HB 9742), (HB 9743), (HB 9744) and (HB 9745).

6. Method as claimed in one or more of the preceding claims, wherein the acid treatment is followed by extraction with organic solvents for samples which are inorganic in nature and extraction with organic solvents for readily soluble samples which are primarily organic in nature.

7. Method as claimed in one or more of the preceding claims, wherein the dioxin fraction is separated via chromatography.

8. Method as claimed in claim 7, wherein the dioxin fraction is separated by solid-liquid chromatography.

9. Method as claimed in claims 7 or 8, wherein said chromatography step uses a matrix or matrices from the group consisting essentially of: carbon-glass, activated silica, oleum-silica, silver nitrate silica and acid alumina.

10. Method as claimed in claims 7, 8 or 9, wherein one or more dyes, having a similar absorption and elution behavior as the substance to be determined, are employed to monitor said chromatography separation step.

11. Method as claimed in one or more of the preceding claims, wherein said dioxin fraction is solubilized into an aqueous buffer solution by addition of one or more detergents.

12. Method as claimed in one or more of the preceding claims, wherein the solubilization of the dioxin fraction into aqueous solution is carried out by ultrasonic mixing.

13. Method as claimed in one or more of the preceding claims, wherein the determination reaction is carried out in a competitive procedure.

14. Method as claimed in claim 13, wherein said determination reaction is indicated by a signal selected from the group comprising: a radioactive isotope emission, a chemiluminescence emission, a fluorescence emission and an enzyme-linked signal.

15. Method as claimed in claims 13 or 14, wherein the competition procdure is carried out with coating antigen concentration of about 1-10 ng per reaction well.

16. Method as claimed in claims 13, 14 or 15, wherein said determination reaction is indicated by an enzyme-linked signal amplified by an avidin-biotin linkage.

17. A test kit for determination of dioxins and dibenzofurans in a sample which contains interfering substances, comprising:
a) a unit for decomposition of the sample by acid treatment;
b) a unit for separation of the dioxin fraction from the samples; and
c) a unit for determination of the dioxin compounds by means of dioxin-specific antibodies.

## Patentansprüche

1. Verfahren zur qualitativen und/oder quantitativen Bestimmung von polyhalogenierten, eben gebauten, als Dioxine und Dibenzofurane bezeichneten, polycyclischen Aromaten in einer Probe, umfassend
a) Herstellung eines Rohextraktes durch Zersetzung der Probe durch Behandlung mit Säure;
b) Abtrennung der Fraktion der polyhalogenierten polycyclischen Aromaten mit ebener Struktur, welche als Dioxinfraktion bekannt ist;
c) Löslichmachen dieser Dioxinfraktion in einer wässerigen Lösung: und
d) Analyse der so erhaltenen Dioxinfraktion durch Reaktion mit spezifischen Antikörpern.

2. Verfahren nach Anspruch 1, in welchem die Dioxine und Dibenzofurane in industriellen Proben bestimmt werden.

3. Verfahren nach Anspruch 1, in welchem die Dioxine und Dibenzofurane in Umweltproben bestimmt werden.

4. Verfahren nach einem oder mehreren der vorausgehenden Ansprüche, in welchem die spezifischen Antikörper monoklonale Antikörper sind.

5. Verfahren nach Anspruch 4, in welchem die spezifischen Antikörper aus Hybridomas der Gruppe, welche DD-1, DD-3, DD-4, DD-5 oder DD-6 mit den ATTC-Zugriffsnummern (HB 9741), (HB 9742), (HB 9743), (HB 9744) und (HB 9745) umfaßt ausgewählt sind.

6. Verfahren nach einem oder mehreren der vorausgehenden Ansprüche, in welchem der Behandlung mit Säure für Proben welche anorganischer Natur sind, eine Extraktion mit organischen Lösungsmitteln, und für leicht lösliche Proben, welche in erster Linie organischer Natur sind, eine Extraktion mit organischen Lösungsmitteln folgt.

7. Verfahren nach einem oder mehreren der vorausgehenden Ansprüche, in welchem die Dioxinfraktion durch Chromatographie abgetrennt wird.

8. Verfahren nach Anspruch 7, in welchem die Dioxinfraktion durch Fest-Flüssig-Chromatographie abgetrennt wird.

9. Verfahren nach einem der Ansprüche 7 oder 8, in welchem in dieser Chromatographiestufe eine Grundsubstanz oder Grundsubstanzen aus der im wesentlichen aus Kohle-Glas, aktivierter Kieselsäure, Oleum-Kieselsäure, Silbernitrat-Kieselsäure und saurem Aluminiumoxid bestehenden Gruppe verwendet wird.

10. Verfahren nach einem der Ansprüche 7, 8 oder 9, in welchem ein oder mehrere Farbstoffe verwendet werden, die ein ähnliches Absorptions- und Elutionsverhalten haben wie die zu bestimmende Substanz, um den Verfahrensschritt der Abtrennung durch Chromatographie zu überwachen.

11. Verfahren nach einem oder mehreren der vorausgehenden Ansprüche, in welchem die Dioxinfraktion durch Zugabe eines oder mehrerer Detergentien in einer wässerigen Pufferlösung löslich gemacht wird.

12. Verfahren nach einem oder mehreren der vorausgehenden Ansprüche, in welchem das Löslichmachen der Dioxinfraktion in wässeriger Lösung durch Vermischen unter Einwirkung von Ultraschall durchgeführt wird.

13. Verfahren nach einem oder mehreren der vorausgehenden Ansprüche, in welchem die Bestimmungsreaktion in einer konkurrierenden Arbeitsweise durchgeführt wird.

14. Verfahren nach Anspruch 13, in welchem die Bestimmungsreaktion durch ein Signal angezeigt wird das aus der Gruppe ausgewählt ist, welche Emission eines radioaktiven Isotops, Emission von Chemilumineszenz, Emission von Fluoreszenz und ein mit einem Enzym gekoppeltes Signal umfaßt.

15. Verfahren nach einem der Ansprüche 13 oder 14, in welchem die konkurrierende Arbeitsweise mit einer Konzentration eines umhüllenden Antigens von etwa 1 - 10 ng pro Reaktionsgefäß durchgeführt wird.

16. Verfahren nach einem der Ansprüche 13, 14 oder 15, in welchem die Bestimmungsreaktion durch ein mit einem Enzym gekoppeltes Signal angezeigt wird, das durch eine Avidin-Biotin-Kopplung verstärkt wird.

17. Analysenkoffer (Test-Kit) zur Bestimmung von Dioxinen und Benzofuranen in einer belästigende Substanzen enthaltenden Probe, umfassend:
a) eine Einheit zum Zersetzen der Probe durch Behandlung mit Säure;
b) eine Einheit zur Abtrennung der Dioxinfraktion von den Proben: und
c) eine Einheit zur Bestimmung der Dioxinverbindungen durch dioxinspezifische Antikörper.

## Revendications

1. Procédé pour la détermination qualitative et/ou quantitative de composés aromatiques polycycliques polyhalogénés plans, désignés par dioxines et dibenzofurannes, dans un échantillon, comprenant :
a) la préparation d'un extrait brut par décomposition de l'échantillon par un traitement à l'aide d'un acide,
b) la séparation de la fraction constituée des composés aromatiques polycycliques polyhalogénés à structure plane, connue sous le nom de fraction dioxine,
c) la solubilisation de ladite fraction dioxine dans une solution aqueuse, et
d) l'analyse de la fraction dioxine ainsi obtenue au moyen d'une réaction avec des anticorps spécifiques.

2. Procédé selon la revendication 1, dans lequel on détermine les dioxines et les dibenzofurannes dans des échantillons industriels.

3. Procédé selon la revendication 1, dans lequel on détermine les dioxines et les dibenzofurannes dans des échantillons prélevés dans l'environnement.

4. Procédé selon l'une quelconque des revendications précédentes, dans lequel les anticorps spécifiques sont des anticorps monoclonaux.

5. Procédé selon la revendication 4, dans lequel les anticorps spécifiques sont choisis parmi les hybridomes du groupe comprenant DD-1, DD-3, DD-4, DD-5 et DD-6, dont les numéros d'entrée ATCC sont (HB 9741), (HB 9742), (HB 9743), (HB 9744) et (HB 9745).

6. Procédé selon l'une quelconque des revendications précédentes, dans lequel le traitement à l'aide d'un acide est suivi d'une extraction par des solvants organiques, dans le cas d'échantillons qui sont de nature minérale, et d'une extraction par des solvants organiques, dans le cas d'échantillons facilement solubles qui sont essentiellement de nature organique.

7. Procédé selon l'une quelconque des revendications précédentes, dans lequel la fraction dioxine est séparée par chromatographie.

8. Procédé selon la revendication 7, dans lequel la fraction dioxine est séparée par chromatographie solide-liquide.

9. Procédé selon la revendication 7 ou 8, dans lequel ladite étape chromatographique utilise une matrice ou des matrices prises dans le groupe constitué essentiellement de carbone-verre, silice activée, oléum-silice, nitrate d'argent-silice et alumine acide.

10. Procédé selon l'une quelconque des revendications 7 à 9, dans lequel on utilise un ou plusieurs colorants, ayant un comportement en absorption et en élution semblable à celui de la substance à déterminer, pour suivre le déroulement de ladite étape de séparation par chromatographie.

11. Procédé selon l'une quelconque des revendications précédentes, dans lequel ladite fraction dioxine est solubilisée dans une solution tampon aqueuse par addition d'un ou de plusieurs tensioactifs.

12. Procédé selon l'une quelconque des revendications précédentes, dans lequel la solubilisation de la fraction dioxine dans une solution aqueuse est réalisée par mélange à l'aide d'ultrasons.

13. Procédé selon l'une quelconque des revendications précédentes, dans lequel la réaction de détermination est effectuée suivant une technique par compétition.

14. Procédé selon la revendication 13, dans lequel le résultat de ladite réaction de détermination est indiqué par un signal choisi dans le groupe comprenant : l'émission d'un isotope radioactif, l'émission d'une chimiluminescence, l'émission d'une fluorescence et un signal lié à une enzyme.

15. Procédé selon la revendication 13 ou 14, dans lequel la technique par compétition est réalisée avec une concentration d'antigène de revêtement d'environ 1 à 10 ng par puits de réaction.

16. Procédé selon l'une quelconque des revendications 13 à 15, dans lequel le résultat de ladite réaction de détermination est indiqué par un signal lié à une enzyme, amplifié par une liaison avidine-biotine.

17. Ensemble pour essai, destiné à la détermination de dioxines et de dibenzofurannes dans un échantillon contenant des substances gênantes, comprenant :
a) une unité pour la décomposition de l'échantillon par traitement au moyen d'un acide,
b) une unité pour la séparation de la fraction dioxine des échantillons, et
c) une unité pour la détermination des composés du type dioxine au moyen d'anticorps spécifiques de dioxine.
